(19) Europäisches Patentamt
European Patent Office
Office européen des brevets

(11) EP 1 786 759 B1

(12) **FASCICULE DE BREVET EUROPEEN**

(45) Date de publication et mention de la délivrance du brevet:
**04.07.2012 Bulletin 2012/27**

(21) Numéro de dépôt: **05798253.0**

(22) Date de dépôt: **19.08.2005**

(51) Int Cl.:
***C07C 251/44*** ***(2006.01)*** ***C07C 49/513*** ***(2006.01)***
***A61K 31/15*** ***(2006.01)*** ***A61K 31/122*** ***(2006.01)***
***A61P 25/28*** ***(2006.01)***

(86) Numéro de dépôt international:
**PCT/FR2005/002116**

(87) Numéro de publication internationale:
**WO 2006/027454 (16.03.2006 Gazette 2006/11)**

(54) **NOUVEAUX DERIVES DU 3,5-SECO-4-NOR-CHOLESTANE ET LEURS UTILISATIONS**

NEUE DERIVATE VON 3,5-SECO-4-NORCHOLESTAN UND VERWENDUNG DAMIT

NOVEL DERIVATIVES OF 3,5-SECO-4-NORCHOLESTANE AND USE THEREOF

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **07.09.2004 FR 0409436**

(43) Date de publication de la demande:
**23.05.2007 Bulletin 2007/21**

(73) Titulaire: **Trophos**
**13288 Marseille Cedex 9 (FR)**

(72) Inventeurs:
- **BORDET, Thierry**
**F-13009 Marseille (FR)**
- **DROUOT, Cyrille**
**83300 Draguignan (FR)**

(74) Mandataire: **Galup, Cédric Olivier Nicolas et al**
**Santarelli**
**146 Rue Paradis**
**13294 Marseille Cedex 6 (FR)**

(56) Documents cités:
**WO-A-97/02027** **WO-A-03/007933**
**US-A- 2 883 424**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 786 759 B1

Printed by Jouve, 75001 PARIS (FR)

## Description

La présente invention concerne l'application à titre de médicaments de dérivés du 3,5-seco-4-nor-cholestane, notamment comme neuroprotecteurs par exemple dans les pathologies et les traumatismes liés à la dégénérescence ou à la mort des motoneurones, les compositions pharmaceutiques les renfermant, de nouveaux dérivés et leur procédé de préparation.
Les processus neurodégénératifs sont caractérisés par le dysfonctionnement et la mort des neurones entraînant la perte des fonctions neurologiques médiées par le cerveau (système nerveux central, SNC), la moelle épinière et le système nerveux périphérique (SNP). Ils peuvent résulter, entre autres, de situations pathologiques regroupées sous le terme de maladies ou affections neurodégénératives, de traumatisme, ou d'exposition à des toxines.
Les pathologies les plus importantes qui sont caractérisées par un processus dégénératif sont :

- les maladies chroniques neurodégénératives, héréditaires ou sporadiques, notamment la maladie d'Alzheimer, la maladie de Huntington, la maladie de Parkinson, la sclérose latérale amyotrophique, les amyotrophies spinales, la maladie de Creutzfeldt-Jakob, la sclérose en plaque, l'adrénoleucodystrophie, l'épilepsie, les démences, la schizophrénie, et les syndromes neurologiques associés au SIDA;
- les lésions neuronales liées au vieillissement ;
- les neuropathies périphériques héréditaires ou lésionnelles, comme les maladies de Fabry, de Charcot-Marie-Tooth, de Krabbe, les leucodystrophies, les neuropathies diabétiques et celles induites par les traitements anticancéreux ;
- les traumatismes du cerveau, des nerfs périphériques ou de la moelle épinière;
- les ischémies du cerveau ou de la moelle épinière suite à un accident cérébro-vasculaire, ou induites par un manque d'irrigation sanguine ;
- les dégénérescences, héréditaires, lésionnelles ou liées au vieillissement des neurones sensoriels de la vision, comme les dégénérescences maculaires, les rétinites pigmentaires, ou les dégénérescences du nerf optique induites par les glaucomes ;
- les dégénérescences, héréditaires, traumatiques ou liées au vieillissement des neurones sensoriels de l'ouïe entraînant une diminution ou une perte de l'audition.

Une partie des voies de signalisation affectées dans ces pathologies sont communes à un grand nombre de maladies neurodégénératives. La maladie d'Alzheimer est la démence la plus fréquente. Elle fait apparaître une atrophie du cerveau, une perte neuronale prédominante dans la corne d'Ammon et elle touche aussi les neurones cholinergiques. D'autres pathologies, comme les atrophies lobaires (maladie de Pick, la maladie de Creutzfeld-Jakob), la démence avec corps de Lewy, les démences vasculaires, la maladie de Parkinson sont associées à une mort neuronale importante à l'origine des symptômes de ces démences.
Il n'existe pas actuellement de traitement efficace pour enrayer les dégénérescences neuronales. Une approche thérapeutique pour protéger les neurones de la mort est l'apport de protéines neurotrophiques.
Ces protéines, telles que BDNF (brain-derived neurotrophic factor), CNTF (ciliary neurotrophic factor), NGF (nerve growth factor), GDNF (glia-derived neurotrophic factor) sont synthétisées au cours du développement embryonnaire ou après lésion chez l'adulte. Ces facteurs de croissance favorisent la survie, la maturation et la différentiation des cellules neuronales. De plus, ils inhibent les mécanismes apoptotiques, activent de multiples voies de survie et protègent un grand nombre de populations neuronales. Leur utilisation est proposée dans la plupart des dégénérescences neuronales.
Des composés qui activeraient l'expression de facteurs neurotrophiques ou qui mimeraient l'action de ces facteurs ont un potentiel thérapeutique pour le traitement des syndromes neurodégénératifs.
En particulier, l'apport de molécules neurotrophiques pour le traitement des dégénérescences neuronales vise trois objectifs :

- compenser une carence potentielle en facteurs neurotrophiques liée à un défaut d'apport par les cibles périphériques ou centrales des neurones et/ou un trouble du transport rétrograde de ces facteurs;
- intervenir de façon non spécifique sur des voies biochimiques impliquées dans la cascade dégénérative;
- favoriser les phénomènes compensateurs naturels de croissance dendritique et d'arborisation des terminaisons nerveuses.

Ces composés présenteraient donc un effet bénéfique dans un grand nombre de pathologies en particulier dans les pathologies touchant les systèmes nerveux périphérique et central.
Par ailleurs, dans le cadre ci-dessus, les motoneurones sont des neurones notamment présents dans la moelle épinière et le tronc cérébral. Leur dégénérescence ou leur mort peut conduire à une faiblesse progressive des muscles des membres, puis à une atrophie et éventuellement à une spasticité (c'est à dire une contraction permanente) du muscle.

Les pathologies les plus importantes qui résultent de la dégénérescence et de la mort des motoneurones spinaux et/ou bulbaires sont la sclérose latérale amyotrophique, également connue sous le nom de maladie de Charcot ou encore maladie de Lou Gehrig, et les amyotrophies spinales infantiles, également connues sous les noms de maladie de Werdnig-Hoffmann ou maladie de Kugelberg-Welander.

En outre, on observe une dégénérescence des motoneurones dans les cas de traumatismes avec écrasement et/ou section de la moelle épinière ou des nerfs moteurs périphériques.

Plus généralement, on parle d'amyotrophies spinales pour les maladies où est impliquée la dégénérescence ou la mort des motoneurones de la moelle épinière.

La sclérose latérale amyotrophique (SLA ou ALS pour Amyotrophic Latéral Sclerosis) est une maladie neurodégénérative associée à différents types d'inclusions tels les corps de Lewy et caractérisée par une dégénérescence des motoneurones spinaux et corticaux dont l'issue fatale est parfois associée à une démence frontale. Au cours du développement de l'ALS, les phénomènes dégénératifs se produisent non seulement dans le cerveau mais également dans la moelle épinière et en conséquence dans le muscle, par défaut d'innervation.

Dans l'art antérieur on connaît la demande internationale WO97/02027 publiée le 23 janvier 1997, décrit de nouveaux composés polyamides, les compositions les contenant et leur utilisation dans le traitement des traumatismes neurologique comme les accidents cérébrovasculaires. Cette demande internationale concerne des dérivés de la spermine, de la spermidine et de la putrescine et des dérivés de guanidine de celle-ci, qui conservent l'activité de la polyamine mais présentent moins d'effets secondaires indésirables. Les composés décrits dans cette demande internationale présentent des structures très différentes de celle de la présente invention.

On connaît également la demande internationale WO03/007933, publiée le 30 janvier 2003 qui décrit des composés de type napthoquinone qui peuvent être utilisés pour inhiber l'agrégation de protéines associées aux maladies neurodégénératives. Les composés décrits sont des composés de type vitamine K qui présentent des structures très différentes de celle de la présente invention. On recherche toujours des composés actifs pour lutter contre les affections évoquées ci-dessus.

Or la demanderesse a découvert que des dérivés du 3,5-seco-4-nor-cholestane et notamment le 3,5-seco-4-nor-cholestan-5-one oxime- 3-ol étaient doués de remarquables propriétés neuroprotectrices, particulièrement vis à vis des motoneurones, des neurones du système nerveux central, des nerfs moteurs et périphériques, et donc étaient utiles comme médicaments.

C'est pourquoi la présente invention a pour objet les composés répondant à la formule I

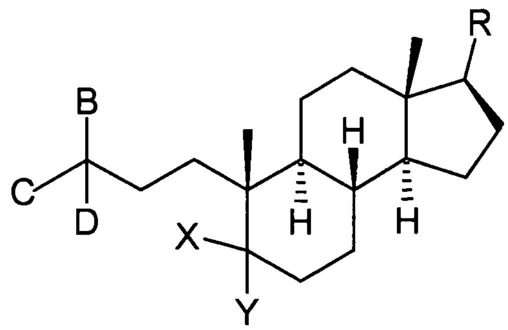

(I)

dans laquelle

- X représente ensemble avec Y une fonction céto, ou X représente un groupement hydroxyle et Y un atome d'hydrogène, ou X et Y représentent ensemble un groupement oxime (=NOH) ou un groupement méthyloxime (=NOMe),
- B représente un groupement hydroxyle et C et D représentent un atome d'hydrogène, ou C et D représentent des radicaux alkyles linéaires ou ramifiés de 1 à 4 atomes de carbone, ou C représente un atome d'hydrogène et D un radical alkyle linéaire ou ramifié de 1 à 4 atomes de carbone,

ou bien B représente ensemble avec C une fonction céto et D un groupement méthyle, hydroxyle, ou méthyl amine
ou bien B et C représentent un atome d'hydrogène et D un groupement méthyl amine.
ou bien B et C ensemble représentent un groupement oxime et D un radical méthyle,
et R représente un radical alkyle linéaire ou ramifié de 1 à 10 atomes de carbone,
leurs esters, ainsi que leurs sels d'addition avec les acides pharmaceutiquement acceptables, pour leur utilisation dans une méthode de traitement thérapeutique du corps humain ou animal, c'est-à-dire à titre de médicaments.

Les sels d'addition avec les acides pharmaceutiquement acceptables peuvent être par exemple des sels formés

avec les acides chlorhydrique, bromhydrique, nitrique, sulfurique, phosphorique acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques, ou carboxyliques.

Comme le comprend l'homme de l'art, un certain nombre de composés de formule 1 qui comprennent un ou plusieurs groupements hydroxyles, peuvent être estérifiés. Ces esters ainsi que leurs sels d'addition avec les acides pharmaceutiquement acceptables ne sont généralement pas directement actifs en eux-mêmes mais constituent des prodrogues pour les analogues hydroxylés correspondants. Ces esters, qui sont métabolisés dans l'organisme humain, conduisent aux composés actifs. Ces esters sont également l'objet de la présente invention. On peut citer les esters introduisant des fonctionnalités chimiques comme les sulfates, les phosphates, les acides et les chaînes basiques qui augmentent la solubilité aqueuse et la biodisponibilité. On préfère les esters de composés portant une fonction basique comme les analogues de dialkylglycine avec des alkyles de 1 à 4 atomes de carbone et tout particulièrement la diméthylglycine et la diéthylglycine et aussi de la méthylpipérazine.

Dans la présente demande et dans ce qui suit, le terme « radical alkyle linéaire ou ramifié de 1 à 4 atomes de carbone désigne par exemple un radical méthyle,éthyle, propyle, isopropyle de préférence un radical méthyle ou éthyle et particulièrement un radical méthyle.

Le terme « radical alkyle linéaire ou ramifié de 1 à 10 atomes de carbone» désigne par exemple un radical 2-méthyle-3-éthyle heptane, 3-éthyle heptane, 3-méthyle heptane , de préférence un radical 2-éthyle heptane et particulièrement le radical 2-méthyle heptane du cholestane, ci-dessous représenté

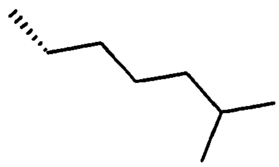

On retient donc plus particulièrement les composés de formule

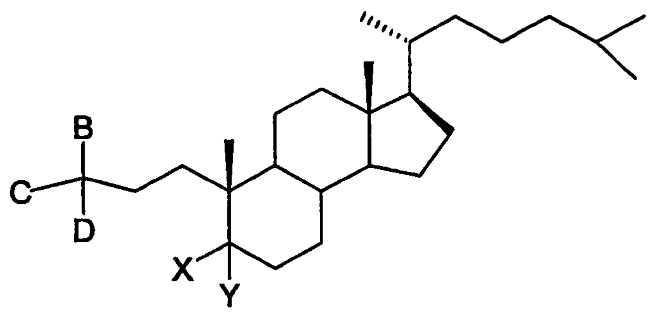

dans laquelle B, C, D, X et Y ont la signification déjà indiquée.

Parmi les composés de formule I ci-dessus décrits, on retient notamment les composés de formule **I** pour lesquels X représente ensemble avec Y une fonction céto ainsi que leurs esters et leurs sels d'addition avec les acides pharmaceutiquement acceptables.

On retient plus particulièrement les composés ci-dessus pour lesquels

- B représente un radical hydroxyle et C et D représentent un atome d'hydrogène, ou C et D représentant 2 radicaux alkyles linéaires ou ramifiés de 1 à 4 atomes de carbone,
- B représente ensemble avec C une fonction céto et D représente un radical méthyle,

ainsi que leurs esters et leurs sels d'addition avec les acides pharmaceutiquement acceptables

Parmi les composés de formule **I** ci-dessus décrits, on retient notamment les composés de formule I pour lesquels X et Y représentent ensemble un groupement oxime, ainsi que leurs esters et leurs sels d'addition avec les acides pharmaceutiquement acceptables.

On retient plus particulièrement les composés ci-dessus pour lesquels

- B représente ensemble avec C une fonction céto et D représente un groupement méthyl, hydroxyle, méthyl amine,
- B représente un groupement hydroxyle, et C et D représentent un atome d'hydrogène, ou C et D représentent 2

radicaux alkyles linéaires ou ramifiés de 1 à 4 atomes de carbone, ou C représente un atome d'hydrogène et D un radical alkyle linéaire ou ramifié de 1 à 4 atomes de carbone,

- B et C représentent un atome d'hydrogène et D un groupement méthyle amine.
- B ensemble avec C représentent un groupement oxime, et D représente un radical méthyle,

ainsi que leurs esters et leurs sels d'addition avec les acides pharmaceutiquement acceptables.

On retient tout particulièrement

- le 3,5-seco-4-nor-cholestan-5-one oxime- 3-ol,
- le 3,5-seco-4-nor-cholestan-5-one oxime-3-méthyl alcool,
- le 3,5-seco-4-nor-cholestan-5-one oxime-3-diméthyl alcool,

ainsi que leurs esters et leurs sels d'addition avec les acides pharmaceutiquement acceptables.

On retient ainsi particulièrement les sels d'additions avec les acides pharmaceutiquement acceptables des composés de formule I ou familles de composés ci-dessus, leurs esters et les sels d'additions avec les acides pharmaceutiquement acceptables desdits esters.

Les composés objet de la présente invention possèdent de très intéressantes propriétés pharmacologiques. Ils sont doués notamment de remarquables propriétés neuroprotectrices, particulièrement vis à vis des motoneurones.

Ces propriétés sont illustrées ci-après dans la partie expérimentale. Elles justifient l'utilisation des composés ci-dessus décrits ainsi que de leurs sels d'addition avec les acides pharmaceutiquement acceptables à titre de médicament.

Les médicaments selon la présente invention trouvent leur emploi en raison de leurs propriétés neuroprotectrices par exemple dans le traitement ou à la prévention des affections neurodégénératives, comme par exemple la maladie de Huntington, les maladies chroniques neurodégénératives, héréditaires ou sporadiques, les lésions neuronales liées au vieillissement, les neuropathies périphériques héréditaires ou lésionnelles, la maladie de Charcot-Marie-Tooth, les neuropathies diabétiques ou induites par les traitement anticancéreux, les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, et notamment les amyotrophies spinales, la sclérose latérale amyotrophique et les pathologies dues aux traumatismes de la moelle épinière ou des nerfs moteurs périphériques.

Dans le contexte de l'invention, le terme « traitement » désigne le traitement préventif, curatif, palliatif, ainsi que la prise en charge des patients (réduction de la souffrance, amélioration de la durée de vie, ralentissement de la progression de la maladie), etc. Le traitement peut en outre être réalisé en combinaison avec d'autres ingrédients ou traitements, tels que notamment d'autres composés actifs pour traiter les pathologies ou traumatismes spécifiés dans la présente demande.

Ils trouvent notamment en raison de leurs propriétés neuroprotectrices vis à vis des motoneurones, leur emploi particulièrement dans le traitement des amyotrophies spinales, notamment de la sclérose latérale amyotrophique ou des amyotrophies spinales infantiles, et dans le traitement des traumatismes de la moelle épinière ou des nerfs moteurs périphériques comme évoqué ci-dessus.

En général la dose journalière du composé sera la dose minimum pour obtenir l'effet thérapeutique. Cette dose dépendra des différents facteurs cités auparavant. Les doses des composés de ci-dessus décrits et par exemple du 3,5-seco-4-nor-cholestan-5-one oxime- 3-ol seront en général comprises entre 0,001 à 100 mg par kilo par jour pour l'homme.

Si nécessaire, la dose journalière peut être administrée en deux, trois, quatre, cinq, six ou plus, prises par jour ou par sous-doses multiples administrées par intervalles appropriés pendant la journée.

La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.

La prescription du médecin traitant pourra commencer à des doses inférieures à celles généralement utilisées, puis ces doses seront progressivement augmentées afin de mieux maîtriser l'apparition d'éventuels effets secondaires.

L'invention a aussi pour objet les compositions pharmaceutiques qui renferment au moins un composé précité ou un de ses sels d'addition avec les acides pharmaceutiquement acceptables, à titre de principe actif.

Dans ces compositions, le principe actif est avantageusement présent à des doses physiologiquement efficaces ; les compositions précitées renferment notamment une dose neuroprotectrice efficace d'au moins un principe actif ci-dessus.

A titre de médicaments, les composés répondant à la formule I, leurs esters, leurs sels d'addition avec les

acides pharmaceutiquement acceptables ainsi que et les sels d'additions avec les acides pharmaceutiquement acceptables desdits esters peuvent être incorporés dans des compositions pharmaceutiques destinées à la voie digestive ou parentérale.

Les compositions pharmaceutiques selon l'invention peuvent comprendre en outre au moins un autre ingrédient thérapeutiquement actif, pour une utilisation simultanée, séparée ou étalée dans le temps, notamment lors d'un traitement chez un sujet atteint d'une pathologie ou d'un traumatisme lié à la dégénérescence ou à la mort des motoneurones tel que défini ci-dessus.

Les compositions pharmaceutiques ou médicaments selon l'invention comprennent avantageusement un ou plusieurs excipients ou véhicules inertes, c'est à dire pharmaceutiquement inactifs et non toxiques. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, les cyclodextrines, le polysorbate 80, le mannitol, la gélatine, le lactose, des huiles végétales ou animales, l'acacia, etc. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

L'administration peut être réalisée par toute méthode connue de l'homme du métier, de préférence par voie orale ou par injection, typiquement par voie intra-péritonéale, intra-cérébrale, intra-thécale, intra-veineuse, intra-artérielle ou intra-musculaire. L'administration par voie orale est préférée. S'agissant d'un traitement à long terme, la voie d'administration préférée sera sublinguale, orale ou transcutanée.

Pour les injections, les composés sont généralement conditionnés sous forme de suspensions liquides, qui peuvent être injectées au moyen de seringues ou de perfusions, par exemple. Il est entendu que le débit et/ou la dose injectée, ou de manière générale la dose à administrer, peuvent être adaptés par l'homme du métier en fonction du patient, de la pathologie, du mode d'administration, etc.. Il est entendu que des administrations répétées peuvent être réalisées, éventuellement en combinaison avec d'autres ingrédients actifs ou tout véhicule acceptable sur le plan pharmaceutique (tampons, solutions saline, isotonique, en présence d'agents stabilisants, etc.).

L'invention est utilisable chez les mammifères, notamment chez l'être humain.

La présente invention a encore pour objet un procédé de préparation d'une composition ci-dessus décrite, caractérisé en ce que l'on mélange, selon des méthodes connues en elles mêmes, le ou les principes actifs avec des excipients acceptables, notamment pharmaceutiquement acceptables.

Les composés de formule I tels que définis ci-dessus sont connus ou peuvent être préparés selon des procédés décrits dans la littérature. Certains dérivés de formule I sont des produits nouveaux.

C'est pourquoi la présente demande a aussi pour objet les composés nouveaux répondant à la formule I

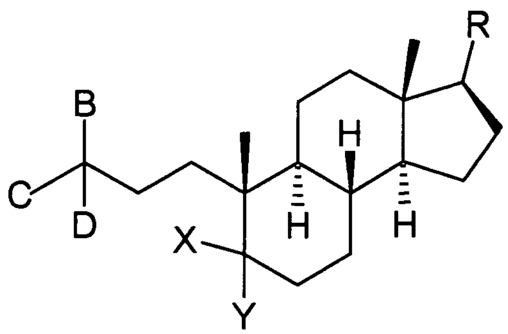

(I)

dans laquelle

- X et Y représentent ensemble un groupement oxime, B et C représentent un atome d'hydrogène et D un groupement méthyl amine.
- X représente ensemble avec Y une fonction céto, B représente un radical hydroxyle et C et D représentant des radicaux méthyle,
- X et Y ensemble représentent un groupement oxime, B représente un radical hydroxyle et C et D représentant des radicaux méthyle,
- X et Y ensemble représentent un groupement oxime, B représente un radical hydroxyle, C représente un atome

d'hydrogène et D représente un radical méthyle,

- X et Y ensemble représentent le groupement méthyl oxime, B représente un radical hydroxyle et C et D représentent des atomes d'hydrogène,

ainsi que leurs sels d'addition avec les acides minéraux ou organiques.

La présente invention a également pour objet un procédé de préparation des nouveaux composés de formule I tels que définis ci-dessus ainsi que de leurs sels, caractérisé en ce que l'on fait réagir un composé de formule II

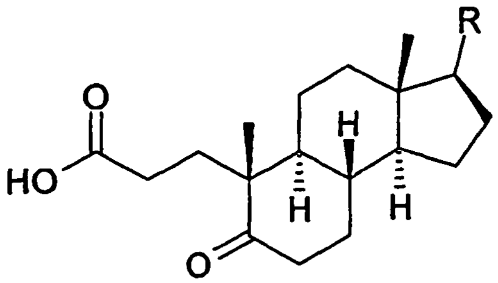

**(II)**

dans laquelle dans laquelle R représente un radical alkyle linéaire ou ramifié de 1 à 10 atomes de carbone, que l'on soumet

- <u>soit</u>
  à l'action de la méthylamine puis de l'hydroxylamine pour obtenir un composé de formule I dans laquelle R a la signification déjà indiquée, X et Y représentent ensemble un groupement oxime, B représente ensemble avec C une fonction céto et D un groupement méthyl amine
- <u>soit</u>
  à une méthylation pour obtenir un composé de formule III

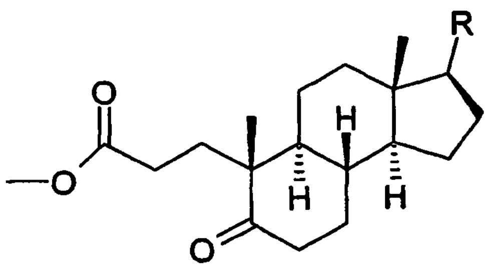

**(III)**

dans laquelle R a la signification déjà indiquée, que l'on soumet à l'action d'un agent de protection de la fonction cétone en position 5 pour obtenir un composé de formule IV

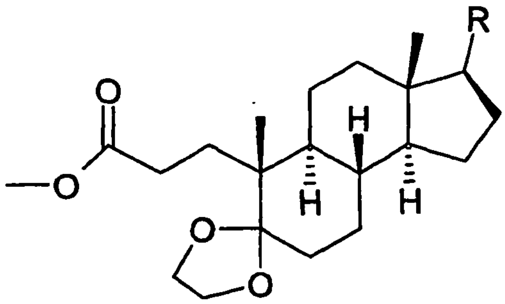

**(IV)**

dans laquelle R a la signification déjà indiquée, que

- <u>ou bien</u>
  l'on fait réagir avec le méthyl lithium puis l'on soumet à l'action d'un agent de déprotection de la fonction cétone en position 5 puis l'on fait réagir avec l'hydroxylamine pour obtenir un composé de formule I dans laquelle R a la

signification déjà indiquée, X et Y représentent ensemble un groupement oxime, B représente un groupement hydroxyle et C et D représentent des radicaux alkyles linéaires ou ramifiés de 1 à 4 atomes de carbone,

- ou bien

l'on saponifie, puis fait réagir avec un composé de formule $H_3C\text{-}NH\text{-}OCH_3$, puis fait réagir avec le méthyl lithium, pour obtenir un composé de formule V

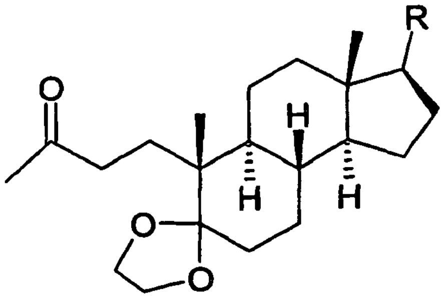

(V)

que l'on soumet à une réduction de la fonction cétone puis l'on soumet à l'action d'un agent de déprotection de la fonction cétone en position 5 puis l'on fait réagir avec l'hydroxylamine pour obtenir un composé de formule I dans laquelle R a la signification déjà indiquée, X et Y représentent ensemble un groupement oxime, B représente un groupement hydroxyle, et C représente un radical alkyle linéaire ou ramifié de 1 à 4 atomes de carbone éventuellement substitué et D représente un atome d'hydrogène,

- ou bien

l'on réduit pour obtenir un composé de formule VI

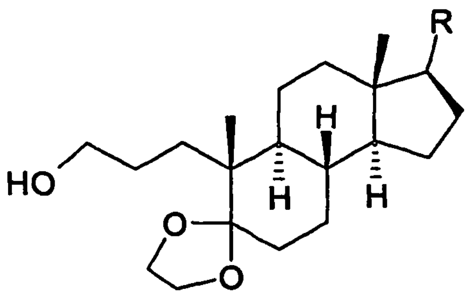

(VI)

dans laquelle R a la signification déjà indiquée, B représente un groupement hydroxyle, et C et D représentent un atome d'hydrogène, que

- soit

l'on soumet à l'action d'un agent d'oxydation pour obtenir un composé de formule VII

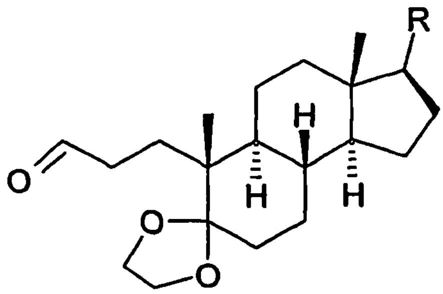

(VII)

dans laquelle R a la signification déjà indiquée dont l'on prépare la base de Schiff puis réduit, puis l'on soumet à l'action d'un agent de déprotection de la fonction cétone en position 5, puis l'on fait réagir avec l'hydroxylamine pour obtenir un composé de formule I dans laquelle R a la signification déjà indiquée, X et Y représentent ensemble un

groupement oxime, B représente un groupement méthylamine, et C et D représentent un atome d'hydrogène,

- soit
  l'on soumet à l'action d'un agent de déprotection de la fonction cétone en position 5, puis l'on fait réagir avec une amine choisie parmi l'hydroxylamine, la méthylhydroxylamine et la carboxyméthylhydroxylamine pour obtenir un composé de formule I dans laquelle R a la signification déjà indiquée, X et Y représentent ensemble respectivement un groupement oxime, méthyloxime, et carboxyméthyloxime, B représente un groupement hydroxyle, et C et D représentent un atome d'hydrogène,
  et l'on isole et si désiré salifie les composés de formule I ou estérifie puis si désiré les composés de formule I.

Dans des conditions préférentielles de mise en oeuvre du procédé ci-dessus décrit,

- La réaction du composé de formule II avec la méthylamine est réalisée en présence d'un agent de couplage activant la fonction acide comme le BOP (Benzotriazole-1-yl-oxy-tris-(diméthylamino)-phosphonium hexafluorophosphate) ou le TBTU ( 2-(1H-benzotriazole-1-yl)-1,1,3,3-tétraméthyluronium tétrafluoroborate) avantageusement en présence d'une base comme la N-méthylmorpholine, notamment dans un solvant adapté comme le dichlorométhane ou le diméthylformamide. De préférence on la réalise en présence de EDCI (1-Ethyl-3-(3'-diméthylaminopropyl)carbodimide) associé à la 4-diméthylaminopyridine dans le dichlorométhane, le mélange étant soumis à une agitation à température ambiante pendant 24 h. Le produit est ensuite mis en solution de préférence dans la pyridine, puis on ajoute de 5 à 7 et notamment 6 équivalents de chlorhydrate d'hydroxylamine.
- La méthylation du composé de formule II est réalisée par réaction avec le méthanol en présence de chlorure de thionyle, de préférence en solubilisant l'acide de formule II dans un volume adapté d'un mélange de 70% de méthanol et 30% de dichlorométhane. On refroidit à 0°C et l'on ajoute goutte-à-goutte 3 équivalents de chlorure de thionyle. On agite alors 2 heures à température ambiante.

Sur ce composé, la protection de la fonction cétone est effectuée de préférence en solubilisant le produit dans un excès, par exemple 10 équivalents de triméthylorthoformate et un volume suffisant d'éthylène glycol, puis addition d'acide p-toluènesulfonique anhydre.

- La réaction du composé de formule IV avec le méthyl lithium est de préférence effectuée dans le THF anhydre puis après refroidissement à environ - 45 °C, addition goutte-à-goutte d'un excès de méthyl lithium.

La déprotection du dioxolane qui bloque la fonction cétone en position 5, est réalisée dans l'acétone en présence d'acide sulfurique. De préférence on l'effectue dans du dioxane, en présence d'un mélange eau /acide acétique 1/1. L'oxime de la cétone est avantageusement réalisée comme ci-dessus.

- La saponification du composé de formule IV est réalisée avec la soude de préférence dans le dioxane. On ajoute notamment environ 2 équivalents d'une solution aqueuse de soude.

Ce produit est mis en réaction avec un composé de formule $H_3C$-NH-$OCH_3$ par exemple en présence d'un agent de couplage activant la fonction acide comme le BOP ou le TBTU en présence d'une base comme la N-méthylmorpholine dans un solvant adapté comme le dichlorométhane ou le diméthylformamide. De préférence on la réalise en présence de EDCI associé à l'hydroxybenzotriazole avec de la triéthylamine ajoutée goutte-à-goutte dans le solvant.
Ce produit est mis en réaction avec le méthyl lithium sous atmosphère d'argon selon le protocole décrit ci-dessus, puis la fonction cétone en 3 est réduite par le borohydrure de sodium.
Le produit obtenu est ensuite soumis à la déprotection de la fonction cétone en position 5 et mis en réaction avec l'hydroxylamine selon le même protocole décrit ci-dessus.

- La réduction du composé de formule IV pour obtenir le composé de formule VI est réalisée de préférence par de l'hydrure d'aluminium lithium notamment en le plaçant en suspension dans du tétrahydrofurane. On hydrolyse avec précaution par addition d'une solution de sulfate de sodium.
- L'oxydation du composé de formule VI est réalisée à l'aide de chlorochromate de pyridimium.

Sur ce produit on obtient la base de Schiff qui est réduite instantanément notamment en solubilisant sous argon de préférence dans l'éthanol en présence de triéthylamine, de chlorhydrate de méthylamine et de tétraisopropoxyde de titane, puis addition de borohydrure de sodium.
La déprotection de la fonction cétone en position 5 ainsi que la réaction avec l'hydroxylamine est effectué dans les conditions précédemment décrites.
Les composés de formule II sont des dérivés connus, décrits dans la littérature, et sont accessibles commercialement.

L'invention a encore pour objet l'utilisation d'un composé de formule I

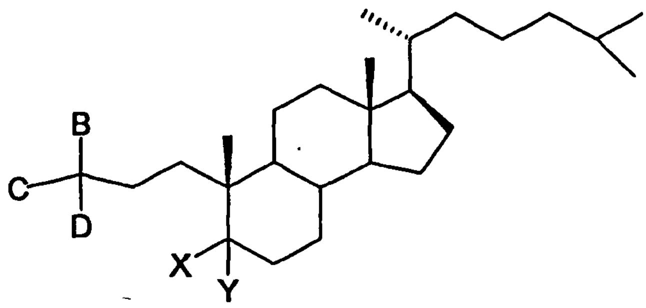

(I)

dans laquelle

- X représente ensemble avec Y une fonction céto, ou X représente un groupement hydroxyle et Y un atome d'hydrogène, ou X et Y représentent ensemble un groupement oxime (=NOH) ou un groupement méthyloxime (=NOMe),
- B représente un groupement hydroxyl et C et D représentent un atome d'hydrogène, ou C et D représentent 2 radicaux alkyles linéaires ou ramifiés de 1 à 4 atomes de carbone, ou C représente un atome d'hydrogène et D un radical alkyle linéaire ou ramifié de 1 à 4 atomes de carbone,

ou bien B représente ensemble avec C une fonction céto et D un groupement méthyle, hydroxyle, ou méthyl amine ou bien B et C représentent un atome d'hydrogène et D un groupement méthyl amine.
ou bien B et C ensemble représentent un groupement oxime et D un radical méthyle,
ou de l'un de ses esters ou de l'un de ses sels d'additions avec les acides pharmaceutiquement acceptables, ou de l'un des sels d'addition de l'un de ses esters avec les acides pharmaceutiquement acceptables, pour l'obtention d'un médicament neuroprotecteur, notamment destiné au traitement des maladies neurodégénératives comme par exemple la maladie de Huntington, les maladies chroniques neurodégénératives, héréditaires ou sporadiques, les lésions neuronales liées au vieillissement, les neuropathies périphériques héréditaires ou lésionnelles, la maladie de Charcot-Marie-Tooth, les neuropathies diabétiques ou induites par les traitement anticancéreux, les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, les maladies et traumatismes liés à la dégénérescence des motoneurones et plus particulièrement les amyotrophies spinales particulièrement infantiles, la sclérose latérale amyotrophique, la sclérose en plaques et les traumatismes de la moelle épinière ou des nerfs moteurs périphériques.

L'invention a particulièrement pour objet l'utilisation d'un composé de formule ci-dessus, sels ou esters pour l'obtention d'un médicament neuroprotecteur, notamment destiné au traitement de pathologies ou traumatismes liés à la dégénérescence ou à la mort des neurones, chez des mammifères (en général des patients) atteints de telles pathologies ou traumatismes.

L'invention a plus particulièrement pour objet l'utilisation d'un composé de formule **I** ou de l'un de ses sels ou esters pour l'obtention d'un médicament destiné au traitement des amyotrophies spinales infantiles et les scléroses latérales amyotrophiques.

La mise en oeuvre de ces médicaments comprend habituellement l'administration à ces mammifères d'une quantité thérapeutiquement efficace d'un composé de formule **I** ou de l'un de ses esters et notamment du 3,5-seco-4-nor-cholestan-5-one oxime- 3-ol, en particulier pour augmenter la survie des neurones ou favoriser la croissance axonale.
L'invention a tout autant pour objet une méthode de traitement des maladies, notamment neurodégénératives, précitées et notamment une méthode de traitement de pathologies ou traumatismes liés à la dégénérescence ou à la mort des neurones, chez des mammifères (en général des patients) atteints de telles pathologies ou traumatismes, comprenant l'administration à ces mammifères d'une quantité thérapeutiquement efficace du 3,5-seco-4-nor-cholestan-5-one oxime-3-ol, en particulier pour augmenter la survie des neurones ou favoriser la croissance axonale.
L'invention a de plus pour objet l'utilisation d'un composé de formule I ou de l'un de ses sels ou esters pour l'obtention d'un médicament destiné au traitement d'une des affections ci-dessus décrites et notamment des pathologies ou traumatismes liés à la dégénérescence ou à la mort des motoneurones, chez des mammifères (en général des patients) atteints de tels pathologies ou traumatismes, comprenant l'administration à ces mammifères d'une quantité thérapeutiquement efficace d'un composé de formule **I** ou de l'un de ses sels ou esters, en particulier pour augmenter la survie

des neurones. Plus spécifiquement, les pathologies liées à la dégénérescence ou à la mort des motoneurones sont la sclérose latérale amyotrophique ou les amyotrophies spinales infantiles.
L'invention a pour objet tout autant la mise à disposition de nouveaux dérivés de la 4-cholestèn-3-one que d'autres dérivés de la 4-cholestèn-3-one que ceux pouvant avoir été décrits dans l'état de la technique. Sont donc exclus ceux décrits dans la littérature.

Les conditions préférentielles ci-dessus décrites de mise en oeuvre des médicaments de formule I s'appliquent également aux autres objets de l'invention visés ci-dessus, notamment aux compositions, nouveaux dérivés, utilisations et méthodes de traitement et inversement.

Les exemples qui suivent illustrent la présente demande.

Les temps de rétention ci-après sont exprimés en minutes et centièmes de minute.

La méthode de chromatographie liquide utilisée pour l'ensemble des produits est la suivante :

**Colonne : Macherey-Nagel - Nucleosil® 300-6 C4 - 150x4,6 mm**

**Gradient : eau (+0.05% acide trifluoroacétique) / acétonitrile (+0,05% acide trifluoroacétique)**

t= 0 min : 60% acétonitrile, 40% $H_2O$
t= 6 min : 100% acétonitrile, 0% $H_2O$
t= 11 min : 100% acétonitrile, 0% $H_2O$
t= 13 min : 60% acétonitrile, 40% $H_2O$
t= 15 min : 60% acétonitrile, 40% $H_2O$.

Les conditions d'ionisation pour le spectromètre de masse sont :

Température de la source : 250°C
Tension de cône : 50 V
Capillaire voltage : 3 kV
Rf lens : 0.3 V

<u>Exemple 1 : 3,5-seco-4-nor-cholestane-5-one,oxime-3-methylamide</u>

Stade A:

Dans un premier temps, dans un ballon on introduit 250 mg de 3,5-seco-4-nor-cholestane-5-one,oxime-3-oic acide, 38 mg de chlorhydrate de méthylamine, 250 mg d'EDCI, 100 mg de DMAP et 2,5 mL de dichlorométhane. La solution est agitée 24 heures à température ambiante puis le milieu réactionnel est dilué par ajout de dichlorométhane et lavé avec une solution de bicarbonate de sodium à 10%. La phase organique est séchée sur sulfate de magnésium puis concentrée sous pression réduite. Le résidu obtenu est purifié par flash chromatographie ($CH_2Cl_2$ / MeOH 95/5). On récupère 176 mg de 3,5-seco-4-nor-cholestane-5-one - 3-méthylamide avec un rendement de 68% .

Analyse

RMN-1H (CDCl3) : conforme

Temps de rétention : 4 min 42 centièmes

Pics détectés en spectrométrie de masse : [M+H]+= 418 ; [2M+H]+=835

Stade B :

Ensuite, dans un ballon, on introduit 50 mg de 3,5-seco-4-nor-cholestane-5-one -3-méthylamide, 50 mg de chlorhydrate d'hydroxylamine dans 1 mL de pyridine. On agite 16 heures à température ambiante puis le milieu réactionnel est concentré sous pression réduite. Le résidu obtenu est repris dans un mélange $CH_2Cl_2$ / $H_2O$ ; la phase organique est séparée, lavée à l'eau, séchée sur sulfate de sodium anhydre et concentrée sous pression réduite. On récupère 40,6 mg de 3,5-seco-4-nor-cholestane-5-one,oxime-3-méthylamide avec un rendement de 78%.

Analyse

RMN-1H ($CDCl_3$) : conforme

Temps de rétention : 3 min 70 centièmes

Pics détectés en spectrométrie de masse : [M+H]+= 433 ; [2M+H]+=865

Exemple 2 : 3,5-seco-4-nor-cholestane-5-one-3-dimethyl alcool

Stade A :

Dans un ballon, on solubilise 10,5 g d'acide 3,5-seco-4-nor-cholestane-5-one-3-oique dans 378 mL de méthanol et 146 mL de dichlorométhane. On refroidit à 0°C et l'on ajoute goutte-à-goutte 5,7 mL de chlorure de thionyle. On agite alors 2 heures à température ambiante. Le milieu réactionnel est concentré sous pression réduite, co-évaporé au toluène puis au dichlorométhane. On obtient 10,3 g de 3,5-seco-4-nor-cholestane-5-one-3-méthyl ester avec rendement de 94%. Le produit est utilisé tel quel sans purification.

RMN-1H ($CDCl_3$) : conforme

Temps de rétention : 4 min 69 centièmes

Pic détecté en spectrométrie de masse : $\{M+H\}^+$ = 419 ; [2M+H]+ = 785

Stade B : 3,5-seco-4-nor-cholestane-5,5(éthylène dioxy)-3-méthyl ester

Dans un ballon, on met en solution 9,62 g de 3,5-seco-4-nor-cholestane-5-one-3-méthyl ester dans 25 mL de triméthylorthoformate et 53 mL d'éthylène glycol. On ajoute alors 400 mg (2,3 mmol) d'acide p-toluènesulfonique anhydre puis on agite 1 nuit à température ambiante. On ajoute de l'acétate d'éthyle au milieu réactionnel; On réalise un lavage par une solution à 10% d'hydrogénocarbonate de sodium. La phase organique est séparée, séchée sur sulfate de magnésium anhydre et concentrée sous pression réduite. On obtient 9,95 g de 3,5-seco-4-nor-cholestane-5,5(éthylène dioxy)-3-méthyl ester avec rendement de 93%. Le produit est utilisé tel quel sans purification.

RMN-1H ($CDCl_3$) : conforme

Temps de rétention : 5 min 76 centièmes

Pic détecté en spectrométrie de masse : {M+H}+ = 463

Stade C :

Dans un ballon, on solubilise 300 mg de 3,5-seco-4-nor-cholestane-5,5(éthylène dioxy)-3-méthyl ester dans 5 mL de THF anhydre. Le milieu est refroidi à -45 °C puis on ajoute goutte-à-goutte 1,36 mL d'une solution de méthyllithium 1,6M dans l'éther. Après 30 minutes d'agitation à -45°C. On ajoute quelques gouttes de méthanol au milieu réactionnel et ramène à température ambiante. On reprend dans 20 mL d'éther diéthylique et on lave avec une solution saturée de bicarbonate de sodium, puis avec une solution saturée de chlorure de sodium. La phase organique est séchée sur sulfate de magnésium, puis concentrée sous pression réduite. On obtient 295 mg 3,5-seco-4-nor-cholestane-5,5(éthylène dioxy)-3-diméthyl alcool (PM=462) avec un rendement de 98%.

Temps de rétention : 5 min 56 centièmes

Pics détectés en spectrométrie de masse : [M- ($CH_2OH$-$CH_2OH$ + $H_2O$)+H]+]=401

Stade D :

Dans un ballon, on ajoute 6 mL d'un mélange eau /acide acétique 1/1 et 295 mg de 3,5-seco-4-nor-cholestane-5,5(éthylène dioxy)-3-diméthyl alcool ; on chauffe à reflux pendant 1h30. Après refroidissement, le milieu réactionnel est dilué avec de l'acétate d'éthyle, lavé avec une solution de saturée de chlorure de sodium, puis avec une solution saturée de bicarbonate de sodium. Enfin, la phase organique est séchée sur sulfate de magnésium et concentrée sous pression réduite. Le produit brut obtenu est purifié par flash chromatographie (éther de pétrole / acétate d'éthyle 8/2). On obtient 180 mg 3,5-seco-4-nor-cholestane-5-one-3-diméthyl alcool avec un rendement de 68%.

RMN-1 H ($CDCl_3$) conforme

Temps de rétention : 5 min 08 centièmes

Pics détectés en spectrométrie de masse: [M+H]+= 419 ; [M-H2O+H]+=401 ; [2M+H]+=837

Exemple 3 : 3,5-seco-4-nor-cholestane-5-one,oxime-3-diméthyl alcool.

Dans un ballon, on introduit 1 g du composé de l'exemple 2, 1 g de chlorhydrate d'hydroxylamine dans 53 mL

de pyridine et quelques mL de dichlorométhane pour solubiliser la cétone. On agite 16 heures à température ambiante puis le milieu réactionnel est concentré sous pression réduite. Le résidu obtenu est repris dans un mélange $CH_2Cl_2$/ / $H_2O$ ; la phase organique est séparée, lavée à l'eau, séchée sur sulfate de sodium anhydre et concentrée sous pression réduite. On récupère 814 mg de 3,5-seco-4-nor-cholestane-5-one, oxime-3-diméthyl alcool avec un rendement de 78%.

RMN-1H ($CDCl_3$): conforme

Temps de rétention : 5 min 09 centièmes

Pics détectés en spectrométrie de masse : [M+H]+= 434; [2M+H]+=867

Exemple 4 : 3,5-seco-4-nor-cholestane-5-one,oxime- 3-méthyl alcool,

Stade A :

Dans un ballon, on place 2 g de 3,5-seco-4-nor-cholestane-5,5(éthylène dioxy)-3-méthyl ester dans 26 mL de dioxane. On ajoute 8,6 mL d'une solution de soude 1 N. Le milieu réactionnel est chauffé à reflux pendant 1 h30 et le dioxane est évaporé sous pression réduite. On acidifie la solution obtenue par ajout d'une solution d'acide chlorhydrique 1 N jusqu'à pH=1 et l'on extrait 2 fois au toluène. Les phases organiques sont réunies, séchées sur sulfate de magnésium anhydre et concentrées sous pression réduite. On récupère 1,92 g d'acide 3,5-seco-4-nor-cholestane-5,5(éthylène dioxy)-3-oïque avec un rendement de 99% qui est utilisé sans traitement supplémentaire dans l'étape suivante.

Stade B :

Dans un ballon, on place 1,9 g d'acide 3,5-seco-4-nor-cholestane-5,5(éthylène dioxy)-3-oïque dans 30 mL de dichlorométhane. On ajoute à cette solution 1,06 g d'EDCI, 743 mg d'HOBT, 537 mg de chlorhydrate de N,O-diméthylhydroxylamine puis 1.37 mL de triéthylamine goutte-à-goutte. On agite à température ambiante pendant 16 heures. On ajoute un mélange $CH_2Cl_2/H_2O$ au milieu réactionnel et l'on extrait 3 fois au dichlorométhane. Les phases organiques sont réunies, séchées sur sulfate de magnésium anhydre et concentrées sous pression réduite. Le résidu obtenu est purifié par flash chromatographie ($CH_2Cl_2$/acétate d'éthyle 8/2). On récupère 1,46 g 3,5-seco-4-nor-cholestane-5,5 (éthylène dioxy)-3-(N,N-méthoxy-méthyl) amide avec un rendement de 70%.

RMN-1 H ($CDCl_3$): conforme

Temps de rétention : 5 min 31 centièmes

Pic détecté en spectrométrie de masse : [M+H]+= 492

Stade C :

dans un ballon sous argon, on introduit 1,4 g de 3,5-seco-4-nor-cholestane-5,5(éthylène dioxy)-3-(N,N-méthoxy-méthyl) amide dans 20 mL de tétrahydrofurane anhydre et l'on refroidit à 0°C. On ajoute alors goutte-à-goutte 3,38 mL d'une solution de méthyllithium 1.6M dans l'éther. Le milieu réactionnel est agité 3h40 à 0°C puis on ajoute goutte-à-goutte une solution de 0.72 mL d'acide chlorhydrique concentré dans 7.28 mL d'eau. Le tétrahydrofurane est évaporé sous pression réduite ; la solution aqueuse obtenue est basifiée par ajout de soude 1 N jusqu'à pH=10. On extrait à l'éther diéthylique ; les phases organiques sont réunies, séchées sur sulfate de magnésium anhydre et concentrées sous pression réduite. Le résidu obtenu est purifié par flash chromatographie (éther de pétrole/acétate d'éthyle 9/1). On récupère 930 mg de 3,5-seco-4-nor-cholestane-5,5(éthylène dioxy)-3-méthyl cétone avec un rendement de 73%.

RMN-1 H ($CDCl_3$) : conforme

Temps de rétention : 5 min 65 centièmes

Pic détecté en spectrométrie de masse : [M+H]+= 403

Stade D :

Dans un ballon, on place 119 mg de 3,5-seco-4-nor-cholestane-5,5(éthylène dioxy)-3-méthylcétone du stade C dans 1,5 mL de méthanol. On refroidit à 0°C et l'on ajoute 10 mg de borohydrure de sodium. Le milieu réactionnel est agité à 0°C pendant 1 h puis concentré sous pression réduite. Le résidu est repris dans de l'eau et extrait au dichlorométhane. La phase organique est séchée sur sulfate de magnésium et concentrée sous pression réduite. On récupère 94 mg 3,5-seco-4-nor-cholestane-5,5(éthylène dioxy)-3-méthylalcool avec un rendement de 78%, ce produit est utilisé tel quel.

RMN-1 H ($CDCl_3$) : conforme

Temps de rétention : 5 min 22 centièmes

Pics détectés en spectrométrie de masse : [M+H]+= 387

Stade E :

On opère comme au stade D de l'exemple 2 pour déprotéger la cétone en 5.

Stade F :

Dans un ballon on introduit 121 mg de 3,5-seco-4-nor-cholestane-5-one-3-méthyl alcool, 1,5 mL de pyridine et 121 mg de chlorhydrate d'hydroxylamine. La solution est agitée deux jours à température ambiante. Le milieu réactionnel est concentré sous pression réduite, repris dans l'eau et extrait au dichlorométhane. La phase organique est ensuite lavée à l'eau, puis séchée sur sulfate de magnésium et concentrée sous pression réduite. Le produit ainsi obtenu est purifié par flash chromatographie (éther de pétrole/acétate d'éthyle 9/1). On obtient 66 mg 3,5-seco-4-nor-cholestane-5-one, oxime-3-méthyl alcool avec un rendement de rendement 53%.

RMN-1 H ($CDCl_3$) : conforme

Temps de rétention : 4 min 91 centièmes

Pics détectés en spectrométrie de masse : [M+H]+= 420

<u>Exemple 5 : 3.5-seco-4-nor-cholestane-5-one,oxime-3-méthylamine</u>

Stade A :

Dans un ballon, on place en suspension 615 mg de LiAIH4 dans 57 mL de THF. On refroidit à 0°C et l'on ajoute goutte-à-goutte une solution de 3,0 g de 3,5-seco-4-nor-cholestane-5,5(éthylène dioxy)-3-méthyl ester, dans 57 mL de tétrahydrofurane. On agite alors à 0°C pendant 5h. On hydrolyse avec précaution par ajout d'une solution de sulfate de sodium ; la solution blanche obtenue est agitée 30 minutes, puis filtrée. Le filtrat est concentré sous pression réduite et repris dans de l'eau, extrait avec de l'acétate d'éthyle. La phase organique est séchée sur sulfate de magnésium puis concentrée sous pression réduite. On obtient 2,55 g 3,5-seco-4-nor-cholestane-5,5(éthylène dioxy)-3-ol avec un rendement de 85%, ce produit est utilisé tel quel.

RMN-1 H ($CDCl_3$) : conforme

Temps de rétention : 4 min 82 centièmes

Pics détectés en spectrométrie de masse : [M- (CH2OH-CH2OH + H2O)+H]+= 373

Stade B :

Dans un ballon sous argon, on solubilise 476 mg de 3,5-seco-4-nor cholestane-5,5(éthylène dioxy)-3-ol, dans 7 mL de dichlorométhane, puis on ajoute 189 mg d'alumine neutre et 399 mg de chlorochromate de pyridinium ; on agite à température ambiante pendant 3h30. Le milieu réactionnel est filtré sur Célite®; le filtrat est concentré sous pression réduite. Le résidu obtenu est purifié par flash chromatographie (toluène / acétate d'éthyle 9/1 puis 8/2). On obtient 328 mg 3,5-seco-4-nor-cholestane-5,5(éthylène dioxy)-3-al avec un rendement de 69%.

Temps de rétention : 5 min 57 centièmes

Pics détectés en spectrométrie de masse : [M+H]+= 433

Stade C :

Dans un ballon sous argon, on solubilise 323 mg de 3,5-seco-4-nor-cholestane-5,5(éthylène dioxy)-3-al dans 3 mL d'éthanol, puis on ajoute 209 μL de triéthylamine, 100 mg de chlorhydrate de méthylamine et 444 μL de tétraisopropoxyde de titane. Le milieu réactionnel est agité 6h à température ambiante, puis on ajoute 42,5 mg de borohydrure de sodium. On agite 16 heures à température ambiante. Le milieu réactionnel est filtré et lavé avec du dichlorométhane. Le filtrat est séché sur sulfate de magnésium et concentré sous pression réduite. Le résidu obtenu est purifié par flash chromatographie (dichlorométhane / méthanol 9/1 à 5/5). On obtient 84 mg de 3,5-seco-4-nor-cholestane-5,5(éthylène dioxy)-,oxime-3-méthylamine avec un rendement de 25%.

RMN-1 H ($CDCl_3$): conforme

Temps de rétention : 3 min 93 centièmes

Pics détectés en spectrométrie de masse : [M+H]+= 448

Stade D :

Dans un ballon, on introduit 50 mg de 3,5-seco-4-nor-cholestane-5,5(éthylène dioxy)-3-méthylamine et 976 μL d'un mélange eau/acide acétique 1/1. Le mélange est porté au reflux pendant 6h. Après refroidissement, le milieu

réactionnel est dilué avec de l'acétate d'éthyle et lavé avec une solution saturée de chlorure de sodium puis avec une solution de bicarbonate de sodium à 5%. La phase organique est séchée sur sulfate de magnésium et concentrée sous pression réduite. Le produit obtenu est purifié par flash chromatographie (dichlorométhane / méthanol 95/5) ; on obtient 5 mg 3,5-seco-4-nor-cholestane-5-one-3-méthylamine avec un rendement de 11 %.

RMN-1 H ($CDCl_3$) : conforme

Temps de rétention : 3 min 68 centièmes

Pics détectés en spectrométrie de masse : [M+H]+= 404

Stade E :

Dans un ballon, on introduit 5 mg de 3,5-seco-4-nor-cholestane-5-one-3-méthylamine, 5 mg de chlorhydrate d'hydroxylamine et 287 μL de pyridine. Le mélange est agité 16 heures à température ambiante. On reprend ensuite dans du dichlorométhane et on lave à l'eau. La phase organique est séchée sur sulfate de magnésium et concentrée sous pression réduite. On obtient 5 mg de 3,5-seco-4-nor-cholestane-5-one, oxime-3-méthylamine avec un rendement de91 %.

Temps de rétention : 3 min 66 centièmes

Pics détectés en spectrométrie de masse : [M+H]+= 419

<u>Exemple 6 : 3.5-seco-4-nor-cholestane-5-one,méthyloxime-3-ol</u>

Dans un ballon, on introduit 20 mg de 3,5-seco-4-nor-cholestane-5-one-3-ol, 20 mg de chlorhydrate de O-méthylhydroxylamine dans 1 mL de pyridine. On agite 36h à température ambiante et l'on rajoute 10 mg de chlorhydrate de O-méthylhydroxylamine. On agite à nouveau 16 heures à température ambiante puis le milieu réactionnel est concentré sous pression réduite. Le résidu obtenu est repris dans un mélange $CH_2Cl_2$ / $H_2O$ ; la phase organique est séparée, lavée à l'eau, séchée sur sulfate magnésium anhydre et concentrée sous pression réduite. On obtient 18 mg d'une huile jaune qui est purifiée par flash chromatographie (éther de pétrole / acétate d'éthyle 9/1). On récupère 5,8 mg de 3,5-seco-4-nor-cholestane-5-one,methyloxime-3-ol avec un rendement de 27%.

Analyse

RMN-1H ($CDCl_3$) : conforme

Temps de rétention : 5 min 50 centièmes

Pic détecté en spectrométrie de masse : [M+H]+= 420

<u>Exemple 7 : 3,5-seco-4-nor-cholestane-5-one carboxyméthyloxime-3-ol</u>

Dans un ballon, on introduit 52 mg de cétone, 25 mg d'hémi-chlorhydrate de carboxymethoxylamine dans 0,5 mL de pyridine. On agite 2 jours à température ambiante puis le milieu réactionnel est concentré sous pression réduite. Le résidu obtenu est repris dans un mélange $CH_2Cl_2$ / $H_2O$ ; la phase organique est séparée, lavée à l'eau puis par une solution d'acide chlorhydrique à 2%, séchée sur sulfate de sodium anhydre et concentrée sous pression réduite. Le résidu obtenu est purifié par flash chromatographie (éther de pétrole / acétate d'éthyle 8/2). On obtient 24 mg avec un rendement de 39% de la carboxyméthyloxime.

Analyse

RMN-1 H ($CDCl_3$): conforme

Temps de rétention : 4 min 40 centièmes

Pic détecté en spectrométrie de masse : [M+H]+= 464 ; $[2M+H]^+$=927

<u>Exemple 8</u>

On a préparé une suspension répondant à la formule

| | |
|---|---|
| 3,5-seco-4-nor-cholestan-5-one oxime- 3-ol | 20 mg par ml |
| Excipient : | Emulsion huileuse |

Exemple 9

On a préparé une forme sèche répondant à la formule

| | |
|---|---|
| Chlorydrate de 3,5-seco-4-nor-cholestan-5-one oxime- 3- N,N-diméthylglycine ester | 250 mg |
| Excipient : qsp une gélule terminée à | 750 mg |

Exemple 10 : Prodrogue du 3,5-seco-4-nor-cholestan-5-one oxime- 3-ol: 3,5-seco-4-nor-cholestan-5-one oxime- 3- N, N-diméthylglycine ester

Dans un ballon, on place 509 mg de 3,5-seco-4-nor-cholestan-5-one-3-ol 182 mg de chlorhydrate de N,N-diméthylglycine, 275 mg d'EDCI et 207 mg de DMAP dans 10 à 15 mL de dichlorométhane. On agite à température ambiante pendant 16 heures. On ajoute au milieu réactionnel une solution de bicarbonate de sodium à 5% et l'on extrait au dichlorométhane. Les phases organiques sont réunies, séchées sur sulfate de sodium anhydre et concentrées sous pression réduite. Le résidu obtenu est purifié par flash chromatographie (toluène / acétate d'éthyle 8/2). On récupère 488 mg avec un rendement de 78%.

Analyse

RMN-1H ($CDCl_3$) : conforme
Temps de rétention : 3 min 77 centièmes
Pic détecté en spectrométrie de masse : [M+H]+= 476
Le produit est ensuite engagé dans la réaction suivante :
Dans un ballon, on introduit 488 mg du produit obtenu et 488 mg de chlorhydrate d'hydroxylamine dans 23 mL de pyridine. On agite 16 heures à température ambiante puis le milieu réactionnel est repris dans un mélange $CH_2Cl_2$ / $H_2O$ ; la phase organique est séparée, lavée à l'eau, séchée sur sulfate de sodium anhydre et concentrée sous pression réduite. On récupère 378 mg de l'oxime avec un rendement de 75%. Le produit est ensuite salifié en présence d'une solution d éther acidifiée par une solution d'HCl, pour obtenir le produit sous forme de chlorydrate.

Analyse

RMN-1 H ($CDCl_3$) : conforme
Temps de rétention : 3 min 43 centièmes
Pic détecté en spectrométrie de masse : [M+H]+= 491

Exemple 11 : Prodrogue du 3,5-seco-4-nor-cholestan-5-one,oxime- 3-ol: 3,5-seco-4-nor-cholestan-5-one,oxime-3-(4-methyl-1-piperazine) propanoate ester

Dans un ballon, on place 264 mg de 3,5-seco-4-nor-cholestan-5-one-3-ol, 121 mg d'acide 4-méthyl-1-piperazine propanoïque sous forme de sel de lithium, 1425 mg d'EDCI et 106 mg de DMAP dans 2 à 3 mL de dichlorométhane. On agite à température ambiante pendant 1 nuit. On ajoute de l'eau au milieu réactionnel et l'on extrait au dichlorométhane. Les phases organiques sont réunies, séchées sur sulfate de sodium anhydre et concentrées sous pression réduite. Le résidu obtenu est purifié par flash chromatographie (toluène / acétate d'éthyle 98/2). On récupère 54 mg du produit désiré avec un rendement de 15%.

Analyse

RMN-1 H ($CDCl_3$) : conforme
Temps de rétention : 3 min 66 centièmes
Pic détecté en spectrométrie de masse : [M+H]+= 545
Le produit est ensuite engagé dans la réaction suivante :
Dans un ballon, on introduit 30 mg du produit obtenu et 30 mg de chlorhydrate d'hydroxylamine dans 1.2 mL de pyridine. On agite 5h30 à température ambiante puis le milieu réactionnel est repris dans un mélange CH2Cl2 / H2O ; la phase organique est séparée, lavée à l'eau, séchée sur sulfate de sodium anhydre et concentrée sous pression réduite. On récupère 19 mg de oxime avec rendement de 13%.

Analyse

RMN-1 H ($CDCl_3$) : conforme
Temps de rétention : 3 min 62 centièmes
Pic détecté en spectrométrie de masse : [M+H]+= 560
Le produit est ensuite salifié en présence d'une solution d éther acidifiée par une solution aqueuse d'acide chlorhydrique, pour obtenir le produit sous forme de di-chlorhydrate.

| Composé N° | |
|---|---|
| 1 | Composé de l'exemple 1 |
| 2 | Composé de l'exemple 4 |
| 3 | Composé de l'exemple 3 |
| 4 | Composé de l'exemple 4 |
| 5 | Composé de l'exemple 5 |
| 6 | Composé de l'exemple 6 |
| 7 | Composé de l'exemple 7 |
| 8 | Acide 3,5-seco-4-nor-cholestan-5-one,oxime-3-oique décrit dans Azasteroidal alkaloids. Synthesis of A-nor-B-homo-5-azacholestane. Rodewald, W. J.; Wicha, J. Univ. Warsaw, Bulletin de l'Academie Polonaise des Sciences, Série des Sciences Chimiques (1963), 11(8), 437-41. |
| 9 | 3,5-seco-4-nor-cholestan-3,5-diol décrit dans USA 2 883 424 |
| 10 | 3,5-seco-4-nor-cholestan-5 one-3-ol décrit dans Azasteroidal alkaloids. Synthesis of A-nor-B-homo-5-azacholestane. Rodewald, W. J.; Wicha, J. Univ. Warsaw, Bulletin de l'Académie Polonaise des Sciences, Série des Sciences Chimiques (1963), 11(8), 437-41. |
| 11 | 3,5-seco-4-nor-cholestan-5-one,oxime- 3-ol décrit dans Azasteroidal alkaloids. Synthesis of A-nor-B-homo-5-azacholestane. Rodewald, W. J.; Wicha, J. Univ. Warsaw, Bulletin de l'Académie Polonaise des Sciences, Série des Sciences Chimiques (1963), 11(8), 437-41. |
| 12 | 3,5-seco-4-nor-cholestan-5-one,oxime- 3-one,oxime décrit dans Unusual reactions of steroidal and non-steroidal 1,5-dioximes. Stereochemistry and mechanism of formation of N-hydroxypiperidine analogs. Pradhan, Suresh K.; Akamanchi, Krishnacharya G.; Divakaran, Pulukkunatt P.; Pradhan, Prakash M. Dep. Chem. Technol., Univ. Bombay, Bombay, India. Heterocycles (1989), 28(2), 813-39. |
| 13 | 3,5-seco-4-nor-cholestan-5-one- 3-méthyl cétone décrit dans Oxidation of cyclic hemiacetals into diketones: final steps in the conversion of a triterpenoid ring A into a steroidal enone by a new short route. Uusvuori, Raimo; Hase, Tapio A. Dep. Chem., Helsinki Univ. Technol., Espoo, Finland. Synthetic Communications (1982), 12(14), 1081-8. |
| 14 | Acide 3,5-seco-4-nor-cholestan-5-one-3-oïque décrit dans Azasteroidal alkaloids. Synthesis of A-nor-B-homo-5-azacholestane. Rodewald, W. J.; Wicha, J. Univ. Warsaw, Bulletin de l'Académie Polonaise des Sciences, Série des Sciences Chimiques (1963), 11(8), 437-41. |

1. Effets des composés de formule I sur la survie des motoneurones

Pour mettre en évidence l'action neuroprotectrice des composés de formule I, la demanderesse a étudié leur activité sur un modèle *in vitro* de privation trophique de motoneurones de rats. On pourra se référer utilement à la demande de brevet WO 0142784 de la demanderesse sur la mise en culture des motoneurones de moelle épinière.
La moelle épinière d'embryons E14 de rat est disséquée et la partie ventrale est dissociée par trituration après trypsination. Les motoneurones sont séparés des autres cellules spinales par une méthode connue (Camu et al, 1993, Purification of spinal motoneurons from chicken and rat embryos by immunopanning. In « Immunoselection Strategies for Neural cell culture », Neuroprotocols : A companion to Methods in Neurosciences 2, 191-199 ; Henderson et al., 1993, Neutrophins promote motor neuron survival and are present in embryonic limb bud. Nature 363 (6426) :266-70). Les cellules sont centrifugées sur un gradient de densité. Les motoneurones sont enrichis dans la fraction des grandes cellules (les moins denses). Les cellules de cette fraction sont incubées avec un anticorps anti-p75, un antigène de surface présent sur les motoneurones. Des anticorps secondaires couplés à des billes magnétiques sont ajoutés et le

mélange de cellules est passé à travers une colonne dans un aimant (Arce *et al,* 1999). Seuls les motoneurones sont retenus : leur pureté est de l'ordre de 90%.

Les motoneurones sont ensemencés à faible densité dans des puits de culture sur un substrat de polyornithine-laminine dans un milieu neurobasal supplémenté selon Raoul et al, 1999, Programmed cell death of embryonic motoneurons triggered through the Fas death receptor. J Cell Biol 147(5) :1049-62. Des contrôles négatifs (absence de facteurs trophiques) et positifs (en présence de BDNF (Brain-Derived Neurotrophic Factor) à 1 ng/ml, GDNF (Glial-Derived Neurotrophic Factor) à 1 ng/ml et CNTF (Ciliary Neurotrophic Factor) à 10 ng/ml, commercialisés par la société américaine PEPROTECH, Inc. et la société Sigma-Aldrich, sont inclus dans chaque série.

Les composés à tester sont ajoutés 60 minutes après l'ensemencement et les cultures sont maintenues à 37°C sous 5% de $CO_2$ pendant 3 jours.

Les motoneurones ont une tendance spontanée à mourir en l'absence de facteurs neurotrophiques (Pettmann et Henderson, 1998, Neuronal cell death. Neuron 20(4) :633-47). Après 3 jours, la survie est évaluée par une mesure de fluorescence après incubation des cellules en présence de calcéine qui devient fluorescente dans les cellules vivantes.

Après 3 jours en culture à 37°C, sous 5% de $CO_2$ et en humidité saturante, jusqu'à 50% des motoneurones ensemencés initialement survivent dans le milieu supplémenté en facteurs neurotrophiques, alors que moins de 15% des motoneurones survivent en milieu basal seul.

L'activité des composés à tester a été évaluée par leur capacité à empêcher la mort des motoneurones quand ils sont additionnés au milieu neurobasal en comparaison avec la survie des motoneurones en milieu supplémenté avec des facteurs neurotrophiques.

Les composés de formule I selon l'invention ont montré une activité à une concentration capable de permettre un meilleurs taux de survie des motoneurones dans le milieu basal. Ce taux de survie est exprimé par un chiffre, le ratio. Si le ratio est supérieur à 0, l'effet des composés est positif sur la survie des motoneurones.

Les résultats obtenus sont les suivants :

| Composé n° | Concentration en μM | Ratio |
|---|---|---|
| 1 | 3 | 0.4 |
| 2 | 3 | 0,5 |
| 3 | 3 | 0,7 |
| 4 | 3 | 0,5 |
| 5 | 3 | 0,3 |
| 6 | 3 | 0,4 |
| 7 | 3 | 0,4 |
| 8 | 3 | 0,4 |
| 9 | 1 | 0,4 |
| 10 | 3 | 0,6 |
| 11 | 3 | 0,8 |
| 12 | 3 | 0,7 |
| 13 | 3 | 0,7 |
| 14 | 10 | 0,4 |

De par leur effet trophique sur les motoneurones spinaux, les composés de formule I selon l'invention se montrent donc utiles comme médicament, notamment dans le traitement des amyotrophies, en particulier dans le traitement de la sclérose latérale amyotrophique ou des amyotrophies spinales infantiles, et dans le traitement des traumatismes de la moelle épinière.

2. Effets des composés de formule 1 sur la neuroprotection

Une axotomie du nerf facial est pratiquée sur des ratons nouveaux-nés âgés de 2-3 jours. Les animaux reçoivent les composés n° n°11, 3 et 4 4 heures avant la section unilatérale du nerf puis quotidiennement pendant 5 jours par voie sous-cutanée. Sept jours après la section du nerf, les animaux sont anesthésiés, puis fixés par perfusion intracardiaque de paraformaldéhyde. Le cerveau est alors prélevé, inclus en paraffine. L'analyse histologique de coupes

sériées de 7µm du noyau facial, colorées au cresyl violet, permet de compter le nombre de motoneurones du côté intact ainsi que du côté du nerf sectionné (Casanovas et al., Prevention by lamotrigine, MK-801 and N omega-nitro-L-arginine methyl ester of motoneuron cell death after neonatal axotomy, Neuroscience, 1996, 71, 313-325).

Les résultats obtenus sont les suivants :

La survie des motoneurones du noyau facial chez des rats nouveaux-nés axotomisés et traités par voie orale par les composés n°11, 3 et 4 donnent respectivement une augmentation jusqu'à 40 % à une dose comprise entre 3 et 30 mg/kg, par comparaison au nerf non sectionné, une augmentation jusqu'à 25 % à une dose de 10 mg/kg, une augmentation jusqu'à 30 % à une dose de 30 mg/kg.

Etude toxicologique

Les administrations chez la souris, en particulier des composés *11,3 et 4*, par la voie intra-péritonéale, à une dose de 30 mg/kg/ jour, par traitement avec administration quotidienne pouvant aller jusqu'à 14 jours, n'ont pas montré de toxicité significative.

## Revendications

1. Un composé répondant à la formule **I**

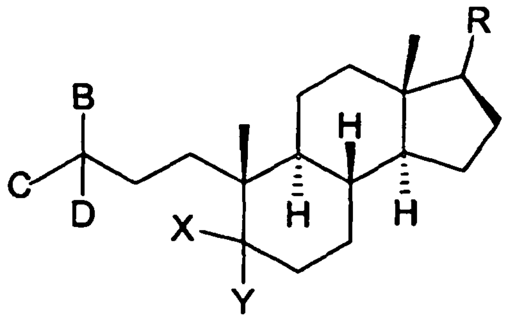

(I)

dans laquelle

- X représente ensemble avec Y une fonction céto, ou X représente un groupement hydroxyle et Y un atome d'hydrogène, ou X et Y représentent ensemble un groupement oxime (=NOH) ou un groupement méthyloxime (=NOMe),
- B représente un groupement hydroxyl et C et D représentent un atome d'hydrogène, ou C et D représentent des radicaux alkyles linéaires ou ramifiés de 1 à 4 atomes de carbone, ou C représente un atome d'hydrogène et D un radical alkyle linéaire ou ramifié de 1 à 4 atomes de carbone,

ou bien B représente ensemble avec C une fonction céto et D un groupement méthyle, hydroxyle, ou méthyl amine ou bien B et C représentent un atome d'hydrogène et D un groupement méthyl amine.
ou bien B et C ensemble représentent un groupement oxime et D un radical méthyle,
et R représente un radical alkyle linéaire ou ramifié de 1 à 10 atomes de carbone,
ou l'un de ses sels d'addition avec les acides pharmaceutiquement acceptables, ou l'un de ses esters ou l'un des sels d'addition avec les acides pharmaceutiquement acceptables desdits esters, pour son utilisation à titre de médicament.

2. Composé selon la revendication 1 **caractérisé en ce que** dans la formule I R représente le radical ci-dessous du cholestane

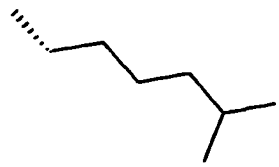

ou l'un de ses sels d'addition avec les acides pharmaceutiquement acceptables, ou l'un de ses esters ou l'un des sels d'addition avec les acides pharmaceutiquement acceptables desdits esters.

3. Composé selon l'une quelconque des revendications 1 ou 2, **caractérisée en ce que** dans la formule I X représente ensemble avec Y une fonction céto ou l'un de ses sels d'addition avec les acides pharmaceutiquement acceptables, ou l'un de ses esters ou l'un des sels d'addition avec les acides pharmaceutiquement acceptables desdits esters.

4. Composé selon l'une quelconque des revendications 1, 2 ou 3,**caractérisée en ce que** dans la formule I B représente un radical hydroxyle et C et D représentent un atome d'hydrogène, ou C et D représentant 2 radicaux alkyles linéaires ou ramifiés de 1 à 4 atomes de carbone, ou l'un de ses sels d'addition avec les acides pharmaceutiquement acceptables, ou l'un de ses esters ou l'un des sels d'addition avec les acides pharmaceutiquement acceptables desdits esters.

5. Composé selon l'une quelconque des revendications 1, 2 ou 3, **caractérisée en ce que** dans la formule I B représente ensemble avec C une fonction céto et D représente un radical méthyle, ou l'un de ses sels d'addition avec les acides pharmaceutiquement acceptables, ou l'un de ses esters ou l'un des sels d'addition avec les acides pharmaceutiquement acceptables desdits esters.

6. Composé selon l'une quelconque des revendications 1 ou 2, **caractérisée en ce que** dans la formule I X et Y représentent ensemble un groupement oxime, ou l'un de ses sels d'addition avec les acides pharmaceutiquement acceptables, ou l'un de ses esters ou l'un des sels d'addition avec les acides pharmaceutiquement acceptables desdits esters.

7. Composé selon la revendication 1 d'au moins un des composés choisi parmi

- le 3,5-seco-4-nor-cholestan-5-one oxime- 3-ol,
- le 3,5-seco-4-nor-cholestan-5-one oxime-3-méthyl alcool,
- le 3,5-seco-4-nor-cholestan-5-one oxime-3-diméthyl alcool,

ou l'un de ses sels d'addition avec les acides pharmaceutiquement acceptables,
ou l'un de ses esters ou l'un des sels d'addition avec les acides pharmaceutiquement acceptables desdits esters.

8. Utilisation d'un composé de formule I selon la revendication 1 ou de l'un de ses sels d'addition avec les acides pharmaceutiquement acceptables, ou de l'un de ses esters ou de l'un des sels d'addition avec les acides pharmaceutiquement acceptables desdits esters, pour l'obtention d'un médicament neuroprotecteur.

9. Utilisation selon la revendication 8 **caractérisée en ce que** le médicament neuroprotecteur est destiné au traitement d'une maladie neurodégénérative.

10. Utilisation selon l'une quelconque des revendications 8 ou 9, **caractérisée en ce que** le médicament neuroprotecteur est destiné au traitement d'une maladie neurodégénérative choisie parmi la maladie de Huntington, les maladies chroniques neurodégénératives, héréditaires ou sporadiques, les lésions neuronales liées au vieillissement, les neuropathies périphériques héréditaires ou lésionnelles, la maladie de Charcot-Marie-Tooth, les neuropathies diabétiques ou induites par les traitement anticancéreux, les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, les maladies et traumatismes liés à la dégénérescence des motoneurones et plus particulièrement les amyotrophies spinales particulièrement infantiles, la sclérose latérale amyotrophique, la sclérose en plaques et les traumatismes de la moelle épinière ou des nerfs moteurs périphériques.

**11.** Utilisation selon l'une quelconque des revendications 8 à 10, **caractérisée en ce que** le médicament neuroprotecteur est destiné au traitement d'une maladie neurodégénérative choisie parmi les pathologies ou traumatismes liés à la dégénérescence ou à la mort des neurones, chez des mammifères atteints de telles pathologies ou traumatismes.

**12.** Utilisation selon l'une quelconque des revendications 8 à 10, **caractérisée en ce que** le médicament neuroprotecteur est destiné au traitement des amyotrophies spinales infantiles.

**13.** Utilisation selon l'une quelconque des revendications 8 à 10, **caractérisée en ce que** le médicament neuroprotecteur est destiné au traitement des scléroses latérales amyotrophiques.

**14.** Utilisation selon l'une des revendications 8 à 13 **caractérisée en ce que** le composé de formule I selon la revendication 1 est le 3,5-seco-4-nor-cholestan-5-one oxime- 3-ol, ou l'un de ses sels d'addition avec les acides pharmaceutiquement acceptables, ou l'un de ses esters ou l'un des sels d'addition avec les acides pharmaceutiquement acceptables desdits esters.

**15.** Un composé répondant à la formule I

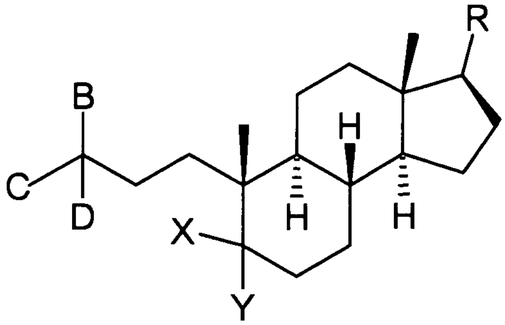

(I)

dans laquelle

- X et Y représentent ensemble un groupement oxime, B et C représentent un atome d'hydrogène et D un groupement méthyl amine.
- X représente ensemble avec Y une fonction céto, B représente un radical hydroxyle et C et D représentant des radicaux méthyle,
- X et Y ensemble représentent un groupement oxime, B représente un radical hydroxyle et C et D représentant des radicaux méthyle,
- X et Y ensemble représentent un groupement oxime, B représente un radical hydroxyle, C représente un atome d'hydrogène et D représente un radical méthyle,
- X et Y ensemble représentent le groupement méthyl oxime, B représente un radical hydroxyle et C et D représentent des atomes d'hydrogène,

et R représente un radical alkyle linéaire ou ramifié de 1 à 10 atomes de carbone,
ou l'un de ses sels d'addition avec les acides pharmaceutiquement acceptables, ou l'un de ses esters ou l'un des sels d'addition avec les acides pharmaceutiquement acceptables desdits esters.

**16.** Une composition pharmaceutique **caractérisée en ce qu'**elle renferme à titre de principe actif l'un au moins des médicaments tels que définis à la revendication 1, ainsi qu'un excipient pharmaceutiquement acceptable.

**17.** Une composition pharmaceutique, **caractérisée en ce qu'**elle renferme à titre de principe actif l'un au moins des médicaments tels que définis à la revendication 2 ainsi qu'un excipient pharmaceutiquement acceptable.

**18.** Une composition pharmaceutique **caractérisée en ce qu'**elle renferme à titre de principe actif l'un au moins des médicaments tels que définis à l'une quelconque des revendications 3 à 7, ainsi qu'un excipient pharmaceutiquement acceptable.

## Claims

1. A compound corresponding to formula I

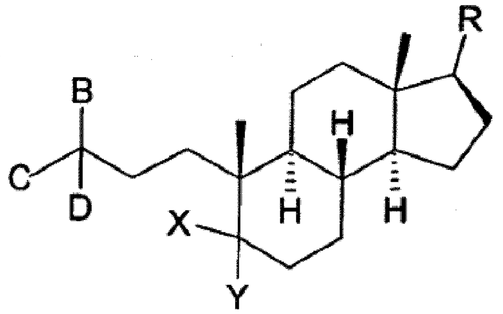

(I)

in which

- X together with Y represents a keto function, or X represents a hydroxyl group and Y a hydrogen atom, or X and Y together represent an oxime group (=NOH) or a methyloxime group (=NOMe),
- B represents a hydroxyl group and C and D represent a hydrogen atom, or C and D represent linear or branched alkyl radicals with 1 to 4 carbon atoms, or C represents a hydrogen atom and D a linear or branched alkyl radical with 1 to 4 carbon atoms,

or B together with C represents a keto function and D a methyl, hydroxyl, or methylamine group
or B and C represent a hydrogen atom and D a methylamine group.
or B and C together represent an oxime group and D a methyl radical,
and R represents a linear or branched alkyl radical with 1 to 10 carbon atoms,
or one of its addition salts with pharmaceutically acceptable acids,
or one of its esters or one of the addition salts with pharmaceutically acceptable acids of said esters, for its use as medicament.

2. Compound according to claim 1, **characterized in that** in formula I R represents the cholestane radical below

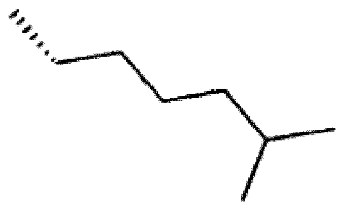

or one of its addition salts with pharmaceutically acceptable acids, or one of its esters or one of the addition salts with pharmaceutically acceptable acids of said esters.

3. Compound according to any one of claims 1 or 2, **characterized in that** in formula I X together with Y represents a keto function or one of its addition salts with pharmaceutically acceptable acids, or one of its esters or one of the addition salts with pharmaceutically acceptable acids of said esters.

4. Compound according to any one of claims 1, 2 or 3, **characterized in that** in formula I B represents a hydroxyl radical and C and D represent a hydrogen atom, or C and D representing 2 linear or branched alkyl radicals with 1 to 4 carbon atoms, or one of its addition salts with pharmaceutically acceptable acids, or one of its esters or one of the addition salts with pharmaceutically acceptable acids of said esters.

5. Compound according to any one of claims 1, 2 or 3, **characterized in that** in formula I B together with C represents a keto function and D represents a methyl radical, or one of its addition salts with pharmaceutically acceptable acids, or one of its esters or one of the addition salts with pharmaceutically acceptable acids of said esters.

6. Compound according to any one of claims 1 or 2, **characterized in that** in formula I X and Y together represent an oxime group, or one of its addition salts with pharmaceutically acceptable acids, or one of its esters or one of the addition salts with pharmaceutically acceptable acids of said esters.

7. Compound according to claim 1, of at least one of the compounds chosen from

   - 3,5-seco-4-nor-cholestan-5-one oxime- 3-ol,
   - 3,5-seco-4-nor-cholestan-5-one oxime-3-methyl alcohol,
   - 3,5-seco-4-nor-cholestan-5-one oxime-3-dimethyl alcohol,

   or one of its addition salts with pharmaceutically acceptable acids,
   or one of its esters or one of the addition salts with pharmaceutically acceptable acids of said esters.

8. Use of a compound of formula I according to claim 1 or one of its addition salts with pharmaceutically acceptable acids, or one of its esters or one of the addition salts with pharmaceutically acceptable acids of said esters, for obtaining a neuroprotective medicament.

9. Use according to claim 8, **characterized in that** the neuroprotective medicament is intended for the treatment of a neurodegenerative disease.

10. Use according to any one of claims 8 or 9, **characterized in that** the neuroprotective medicament is intended for the treatment of a neurodegenerative disease chosen from Huntington's chorea, hereditary or sporadic, chronic neurodegenerative diseases, neuronal lesions linked with ageing; hereditary peripheral neuropathies or peripheral neuropathies resulting from a lesion, Charcot-Marie-Tooth disease, diabetic neuropathies or those induced by anti-cancer treatments, traumatisms of the brain, the peripheral nerves or the spinal cord, ischemias of the brain or the spinal cord, degenerations which are hereditary, result from a lesion or are linked with ageing of the sensory neurons of vision or degenerations of the optic nerve, degenerations which are hereditary, traumatic or linked with ageing of the sensory neurons of hearing, lobar atrophies and vascular dementias, diseases and traumatisms linked to degeneration of the motor neurons and more particularly spinal amyotrophies particularly infantile, amyotrophic lateral sclerosis, multiple sclerosis and traumatisms of the spinal cord or the peripheral motor nerves.

11. Use according to any one of claims 8 to 10, **characterized in that** the neuroprotective medicament is intended for the treatment of a neurodegenerative disease chosen from the pathologies or traumatisms linked to the degeneration or death of the neurons, in mammals suffering from such pathologies or traumatisms.

12. Use according to any one of claims 8 to 10, **characterized in that** the neuroprotective medicament is intended for the treatment of infantile spinal amyotrophies.

13. Use according to any one of claims 8 to 10, **characterized in that** the neuroprotective medicament is intended for the treatment of amyotrophic lateral scleroses.

14. Use according to any one of claims 8 to 13, **characterized in that** the compound of formula I according to claim 1 is 3,5-seco-4-nor-cholestan-5-one oxime-3-ol, or one of its addition salts with pharmaceutically acceptable acids, or one of its esters or one of the addition salts with pharmaceutically acceptable acids of said esters.

15. A compound corresponding to formula I

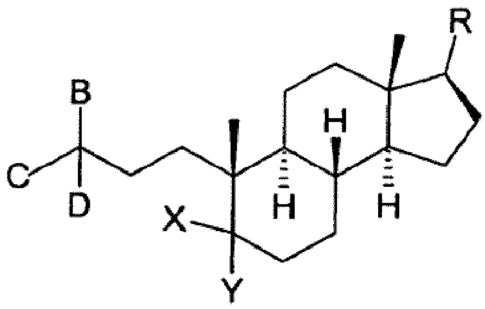

(I)

in which

- X and Y together represent an oxime group, B and C represent a hydrogen atom and D a methylamine group.
- X together with Y represents a keto function, B represents a hydroxyl radical and C and D representing methyl radicals,
- X and Y together represent an oxime group, B represents a hydroxyl radical and C and D representing methyl radicals,
- X and Y together represent an oxime group, B represents a hydroxyl radical, C represents a hydrogen atom and D represents a methyl radical,
- X and Y together represent the methyl oxime group, B represents a hydroxyl radical and C and D represent hydrogen atoms,

and R represents a linear or branched alkyl radical with 1 to 10 carbon atoms,
or one of its addition salts with pharmaceutically acceptable acids, or one of its esters or one of the addition salts with pharmaceutically acceptable acids of said esters.

**16.** A pharmaceutical composition **characterized in that** it contains at least one of the medicaments as defined in claim 1 as active ingredient, as well as a pharmaceutically acceptable excipient.

**17.** A pharmaceutical composition **characterized in that** it contains at least one of the medicaments as defined in claim 2 as active ingredient, as well as a pharmaceutically acceptable excipient.

**18.** A pharmaceutical composition **characterized in that** it contains at least one of the medicaments as defined in any one of claims 3 to 7 as active ingredient, as well as a pharmaceutically acceptable excipient.

**Patentansprüche**

**1.** Verbindung gemäß Formel I

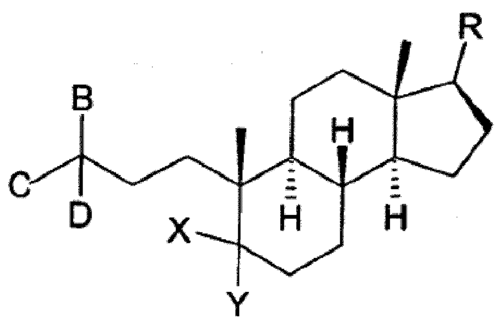

(I)

bei der:

- X zusammen mit Y eine Ketofunktion repräsentiert oder X eine Hydroxylgruppe und Y ein Wasserstoffatom repräsentiert oder X und Y zusammen eine Oximgruppe (=NOH) oder eine Methyloximgruppe (=NOMe) repräsentieren,
- B eine Hydroxylgruppe repräsentiert und C und D ein Wasserstoffatom repräsentieren oder C und D lineare oder verzweigte Alkylradikale mit 1 bis 4 Kohlenstoffatomen repräsentieren oder C ein Wasserstoffatom repräsentiert und D ein lineares oder verzweigtes Alkylradikal mit 1 bis 4 Kohlenstoffatomen repräsentiert,

oder B zusammen mit C eine Ketofunktion repräsentiert und D eine Methyl-, Hydroxyl- oder Methylamingruppe repräsentiert,
oder B und C ein Wasserstoffatom repräsentieren und D eine Methylamingruppe repräsentiert,
oder B und C zusammen eine Oximgruppe repräsentieren und D ein Methylradikal repräsentiert,
und R ein lineares oder verzweigtes Alkylradikal mit 1 bis 10 Kohlenstoffatomen repräsentiert,
oder eines ihrer Additionssalze mit den pharmazeutisch akzeptablen Säuren, oder eines ihrer Ester oder eines ihrer Additionssalze mit den pharmazeutisch akzeptablen Säuren der genannten Ester, zur Verwendung als Medikament.

2. Verbindung nach Anspruch 1, **dadurch gekennzeichnet, dass** in Formel I R das nachfolgende Radikal von Cholestan

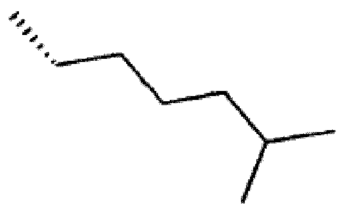

repräsentiert, oder eines ihrer Additionssalze mit den pharmazeutisch akzeptablen Säuren, oder eines ihrer Ester oder eines ihrer Additionssalze mit den pharmazeutisch akzeptablen Säuren der genannten Ester.

3. Verbindung nach Anspruch 1 oder 2, **dadurch gekennzeichnet, dass** in Formel I X zusammen mit Y eine Ketofunktion repräsentiert, oder eines ihrer Additionssalze mit den pharmazeutisch akzeptablen Säuren oder eines ihrer Ester oder eines ihrer Additionssalze mit den pharmazeutisch akzeptablen Säuren der genannten Ester.

4. Verbindung nach Anspruch 1, 2 oder 3, **dadurch gekennzeichnet, dass** in Formel I B ein Hydroxylradikal repräsentiert und C und D ein Wasserstoffatom repräsentieren oder C und D zwei lineare oder verzweigte Alkylradikale mit 1 bis 4 Kohlenstoffatomen oder eines ihrer Additionssalze mit den pharmazeutisch akzeptablen Säuren oder eines ihrer Ester oder eines ihrer Additionssalze mit den pharmazeutisch akzeptablen Säuren der genannten Ester repräsentieren.

5. Verbindung nach Anspruch 1, 2 oder 3, **dadurch gekennzeichnet, dass** in Formel I B zusammen mit C eine Ketofunktion repräsentiert und D ein Methylradikal repräsentiert, oder eines ihrer Additionssalze mit den pharmazeutisch akzeptablen Säuren oder eines ihrer Ester oder eines ihrer Additionssalze mit den pharmazeutisch akzeptablen Säuren der genannten Ester.

6. Verbindung nach Anspruch 1 oder 2, **dadurch gekennzeichnet, dass** in Formel I X und Y zusammen eine Oximgruppe repräsentieren, oder eines ihrer Additionssalze mit den pharmazeutisch akzeptablen Säuren oder eines ihrer Ester oder eines ihrer Additionssalze mit den pharmazeutisch akzeptablen Säuren der genannten Ester.

7. Verbindung nach Anspruch 1 von wenigstens einer der Verbindungen, die ausgewählt sind aus

- 3,5-Seco-4-nor-cholestan-5-on Oxim-3-ol,
- 3,5-Seco-4-nor-cholestan-5-on Oxim-3-methylalkohol,
- 3,5-Seco-4-nor-cholestan-5-on Oxim-3-dimethylalkohol,

oder eines ihrer Additionssalze mit den pharmazeutisch akzeptablen Säuren oder eines ihrer Ester oder eines ihrer Additionssalze mit den pharmazeutisch akzeptablen Säuren der genannten Ester.

8. Verwendung einer Verbindung von Formel I gemäß Anspruch 1 oder eines ihrer Additionssalze mit den pharmazeutisch akzeptablen Säuren oder eines ihrer Ester oder eines ihrer Additionssalze mit den pharmazeutisch akzeptablen Säuren der genannten Ester zum Erhalten eines Neuroprotektormedikaments.

**9.** Verwendung nach Anspruch 8, **dadurch gekennzeichnet, dass** das Neuroprotektormedikament zur Behandlung einer neurodegenerativen Krankheit bestimmt ist.

**10.** Verwendung nach Anspruch 8 oder 9, **dadurch gekennzeichnet, dass** das Neuroprotektormedikament zur Behandlung einer neurodegenerativen Krankheit bestimmt ist, die ausgewählt ist aus der Huntington-Krankheit, chronischen neurodegenerativen, vererbbaren oder sporadischen Krankheiten, altersbedingten neuronalen Läsionen, peripheren vererbbaren oder läsionalen Neuropathien, der Charcot-Marie-Tooth-Krankheit, diabetischen oder durch Krebsbehandlung verursachten Neuropathien, Schäden an Gehirn, peripheren Nerven oder Rückenmark, Ischämien an Gehirn oder Rückenmark, vererbbarer, läsionaler oder altersbedingter Degeneration der sensorischen Neuronen des Sehens oder Degeneration des Sehnervs, vererbbarer, traumatischer oder altersbedingter Degeneration der sensorischen Neuronen des Gehörs, lobären Atrophien und vaskulären Demenzen, Erkrankungen und Schäden in Verbindung mit der Degeneration von Motorneuronen und insbesondere der spinalen, besonders der infantilen Amyotrophien, amyotropher lateraler Sklerose, multipler Sklerose und Schäden an Rückenmark oder peripheren motorischen Nerven.

**11.** Verwendung nach einem der Ansprüche 8 bis 10, **dadurch gekennzeichnet, dass** das Neuroprotektormedikament zur Behandlung einer neurodegenerativen Krankheit bestimmt ist, die ausgewählt ist aus Pathologien oder Schäden in Verbindung mit der Degeneration oder dem Absterben von Neuronen bei Säugetieren, die sich solche Pathologien oder Schäden zugezogen haben.

**12.** Verwendung nach einem der Ansprüche 8 bis 10, **dadurch gekennzeichnet, dass** das Neuroprotektormedikament zur Behandlung von infantilen spinalen Amyotrophien bestimmt ist.

**13.** Verwendung nach einem der Ansprüche 8 bis 10, **dadurch gekennzeichnet, dass** das Neuroprotektormedikament zur Behandlung von amyotrophen lateralen Sklerosen bestimmt ist.

**14.** Verwendung nach einem der Ansprüche 8 bis 13, **dadurch gekennzeichnet, dass** die Verbindung von Formel I gemäß Anspruch 1 3,5-Seco-4-nor-cholestan-5-on Oxim-3-ol oder eines ihrer Additionssalze mit den pharmazeutisch akzeptablen Säuren oder eines ihrer Ester oder eines ihrer Additionssalze mit den pharmazeutisch akzeptablen Säuren der genannten Ester ist.

**15.** Verbindung gemäß Formel I

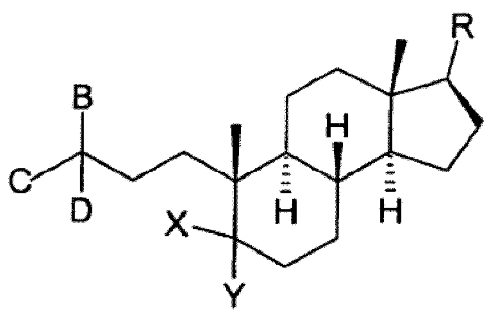

(I)

bei der:

- X und Y zusammen eine Oximgruppe repräsentieren, B und C ein Wasserstoffatom repräsentieren, und D eine Methylamingruppe repräsentiert,
- X zusammen mit Y eine Ketofunktion repräsentiert, B ein Hydroxylradikal repräsentiert und C und D Methylradikale repräsentieren,
- X und Y zusammen eine Oximgruppe repräsentieren, B ein Hydroxylradikal repräsentiert und C und D Methylradikale repräsentieren,
- X und Y zusammen eine Oximgruppe repräsentieren, B ein Hydroxylradikal repräsentiert, C ein Wasserstoffatom repräsentiert und D ein Methylradikal repräsentiert,
- X und Y zusammen die Oximmethylgruppe repräsentieren, B ein Hydroxylradikal repräsentiert und C und D Wasserstoffatome repräsentieren,

und R ein lineares oder verzweigtes Alkylradikal mit 1 bis 10 Kohlenstoffatomen repräsentiert,
oder eines ihrer Additionssalze mit den pharmazeutisch akzeptablen Säuren oder eines ihrer Ester oder eines ihrer Additionssalze mit den pharmazeutisch akzeptablen Säuren der genannten Ester.

**16.** Pharmazeutische Zusammensetzung, **dadurch gekennzeichnet, dass** sie als Wirkstoff wenigstens eines der Medikamente wie in Anspruch 1 definiert sowie einen pharmazeutisch akzeptablen Exzipienten beinhaltet.

**17.** Pharmazeutische Zusammensetzung, **dadurch gekennzeichnet, dass** sie als Wirkstoff wenigstens eines der Medikamente wie in Anspruch 2 definiert sowie einen pharmazeutisch akzeptablen Exzipienten beinhaltet.

**18.** Pharmazeutische Zusammensetzung, **dadurch gekennzeichnet, dass** sie als Wirkstoff wenigstens eines der Medikamente wie in einem der Ansprüche 3 bis 7 definiert sowie einen pharmazeutisch akzeptablen Exzipienten beinhaltet.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9702027 A
- WO 03007933 A
- US 2883424 A
- WO 0142784 A


### Littérature non-brevet citée dans la description


- **RODEWALD, W. J. ; WICHA, J. UNIV. WARSAW.** Bulletin de l'Academie Polonaise des Sciences, Série des Sciences Chimiques. 1963, vol. 11, 437-41
- **RODEWALD, W. J. ; WICHA, J.** Bulletin de l'Académie Polonaise des Sciences, Série des Sciences Chimiques. 1963, vol. 11, 437-41
- **PRADHAN, SURESH K. ; AKAMANCHI, KRISHNACHARYA G. ; DIVAKARAN, PULUKKUNATT P. ; PRADHAN, PRAKASH M.** *Heterocycles,* 1989, vol. 28 (2), 813-39
- **UUSVUORI, RAIMO ; HASE, TAPIO A.** *Synthetic Communications,* 1982, vol. 12 (14), 1081-8
- **CAMU et al.** Purification of spinal motoneurons from chicken and rat embryos by immunopanning. *Immunoselection Strategies for Neural cell culture,* 1993, vol. 2, 191-199
- **HENDERSON et al.** Neutrophins promote motor neuron survival and are present in embryonic limb bud. *Nature,* 1993, vol. 363 (6426), 266-70
- **RAOUL et al.** Programmed cell death of embryonic motoneurons triggered through the Fas death receptor. *J Cell Biol,* 1999, vol. 147 (5), 1049-62
- **PETTMANN ET HENDERSON.** Neuronal cell death. *Neuron,* 1998, vol. 20 (4), 633-47
- **CASANOVAS et al.** Prevention by lamotrigine, MK-801 and N omega-nitro-L-arginine methyl ester of motoneuron cell death after neonatal axotomy. *Neuroscience,* 1996, vol. 71, 313-325